(19) Europäisches Patentamt
European Patent Office
Office européen des brevets

(11) Publication number: 0 114 581
B1

(12) # EUROPEAN PATENT SPECIFICATION

(45) Date of publication of patent specification: 26.08.87

(51) Int. Cl.$^4$: **A 61 L 15/01,** A 61 L 15/03, A 61 K 9/22

(21) Application number: 83830269.3

(22) Date of filing: 16.12.83

(54) **Reactive polymers for dermal and transdermal therapy.**

(30) Priority: 17.12.82 IT 2483982

(43) Date of publication of application:
01.08.84 Bulletin 84/31

(45) Publication of the grant of the patent:
26.08.87 Bulletin 87/35

(84) Designated Contracting States:
AT BE CH DE FR GB LI LU NL SE

(58) References cited:
FR-A-2 124 500
FR-A-2 216 333
US-A-3 526 224

(73) Proprietor: SOGIMI S.r.l.
Via N. Bixio, 2
Fino Mornasco (Como) (IT)

(72) Inventor: Snider, Bruno
Via Monticello 4
Capiago Intimiano (Como) (IT)

(74) Representative: Aimi, Luciano et al
c/o Società Italiana Brevetti S.p.A.
Via Carducci 8
I-20123 Milano (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 114 581 B1

**Description**

This invention relates to reactive polymers for the treatment of skin afflictions and particularly crosslinkable urethane polymers with film forming properties and applicable to various skin disorders, and also to healthy skin as transdermal drugs carriers.

It is known that various treatments of skin diseases or the like have been proposed and used over the years, mostly through topical application of liniments, unguents or caustic agents such as nitric acid, chloroacetic acid, salicyclic acid, etc.

Moreover, for many years, natural or synthetic polymers that have a relative affinity with skin tissues, have been employed as protective agents or as excipients and carriers for dermoreactive substances.

Prior procedures of external medical action, to be classified in the category of liquid plasters for spray application, supply protection films, owing to evaporation of the solvent constituted of water or of water mixed with organic compatible solvents. Other similar products are mixed compositions of polymers and medicinal additives (haemostatics, disinfectants, antibiotics; dermoprotective agents). These prior products consist mostly of copolymers whose duration "in situ" after application, is of very few days, because of their sensitivity to water and perspiration. Their use is anyway suggested for the medication of wounds of small or medium dimensions, and the principal advantage of these materials is often limited to their use. Products belonging to this family have also been obtained with natural polymers of cellulosic kind, more or less chemically modified. Anyway, they are always products having a certain water sensitivity, that adhere to skin only through physical bonds.

In the literature of the field there was also mentioned the use in exceptional cases of chemical unsaturated reactive substances, that, applied on serious wounds, with hemorrhage danger, facilitate the arrest of the blood flow of wounds, due to a violent hemostatic action. These chemical substances are monomers of the cyanoacrylate family, that at skin contact, and by means of its humidity, polymerize, in that they are hydro-reactive products chemically combining in correspondence to wounds.

Anyway, these products are not suitable for the normal epidermal and transdermal skin therapy, because of the absolute lack of elasticity and because of chemical toxicity, negative aspects that could be only accepted in drastic situations (serious wounds in war situations, mutilations, dangers of serious hemorrhages).

Filmogenic polymers, endowed with hydrorepellent action, used in order to isolate recent wounds or sores sensible to the soaking water effect, were recently introduced in the pharmaceutical field. Such are for example alkylpolysiloxanes and other silicone based polymers that deposit upon skin through physical adhesion. They were used in the treatment of bedsores.

More recently tested is the use of a polymer on the basis of animal collagen fibres, alone or in combination, in separate layers, with siliconic polymers, which always constitute the external protective layer.

It has been surprisingly discovered, and this is the object of the present invention, that certain urethane polymers, besides having superior mechanical and chemical characteristics than the polymers known until now for analogous employments, have the property of chemically binding to skin proteins, and more generally to the body proteins, showing a real therapeutic action on the alterations of animal skin, particularly of the human skin. According to the present invention, the principal characteristics of polyurethanes are: dermoreactivity, that is the capacity of closely binding to skin through chemical reaction; elasticity very similar to that of skin; a good resistance to abrasion as well as a good resistance and impermeability to water.

Such polymers, applied to skin as a thin film, completely react with and remain bound to skin until the replacement of the horny layer, thus protecting the underlying derma in the reconstructive phase and accelerating the regeneration through stimulation of the damaged tissues. The resulting polymeric film has elasticity characteristics similar to those of skin, thus following contours and deformations, superior mechanical characteristics, good resistance to the soaking action of water and to the attack of substances of ordinary occasional use that can cause skin troubles, particularly if sensitive, such as detergents, certain cosmetics and the like. The film is usually waterproof having partial air-permeability, but it is possible to modify the formulation to allow a greater ratio of air penetration, and consequently to improve the oxygenation of the deepest skin layers.

The dermoreactive polymers according to the present invention, are constituted of two components that are combined by mixing them immediately before the application on to the skin surface to be treated, and by leaving the applied coating to react for a determined period of time specifically fixed for the application in question. The resultant polymers have a very high molecular weight and are cross-linked with a three dimensional structure, being very resisting even to the most common organic solvents; they steadily adhere to the epidermis and remain thereon for a period of time ranging from few days to three weeks about, that is until the healthy skin layers entirely regenerate. Just then, during the separation of the horny layer, owing to regeneration, the polymer film is removed therewith, leaving the new and healthy skin surface.

The first component A is an oligomer, with hydroxylic, amidic or aminic chemical functions. The second component B is constituted of a monomer, mostly an oligomer with isocyanate reactive chemical

functions. The resultant reaction principally regards hydroxylic, aminic or amidic and isocyanate groups according to the schematic equations well-known in the chemistry.

Another kind of reaction is the one that happens between the isocyanate groups and water, present in the atmosphere leaving the skin damp or in the skin exudate, with formation of new aminic groups, and the liberation of carbon dioxide, helping in this way the cross linking reactions.

The —NCO functions of isocyanates react even with aminic and amidic hydrogens of the polypeptidic chains constituting the skin organic tissues according to the following schematic equation:

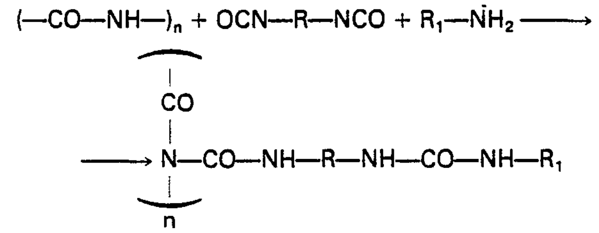

This reaction has a fundamental importance, from the point of view of the present invention, because it determines the polymer adhesion to skin. The polymer's reaction with damaged epidermic layers or anyway with superficial tissues determines a gradual devitalization of unhealthy skin cells, so as to facilitate the body's rejection mechanism and to stimulate the regeneration of healthy cells.

In order to effect exactly the polymer's application, according to the present invention, it is necessary that the part to be treated is clean as possible from fatty substances and dry, so as to facilitate the perfect adhesion and the sealing surface action of the polymer film. For this reason, in the cases of pathologies with the presence of damp surfaces, it is necessary to dry the skin with absorbent materials having a large absorption capacity. For the most difficult cases in this sense, it will be useful to employ a combination of quickly reacting components, so that the polymer in formation quickly binds to the surface and acts as a barrier thus stopping the humor flow.

The pathologic cases in which the product, according to the present invention, has given the most evident results, are the different kinds of eczema or of dermatitis of allergic-toxinic, funginic, bacteric or chemical origin; troubles such as rhagades, chilblains, bedsores, burn sores and burns in general.

Most cases, examined in practical experiments, regard afflictions of chronic character, where therapy of modern medicine and pharmacology did not succeed in curing the disease: these were cases where one had often to deal with cortisone treatments, in order to mitigate temporarily the disease inconvenience, but this, as is known, reduces the efficacy of the immunity system, and anyway does not succeed in healing. The application of this kind of polymer, instead, could provide an effective treatment.

In the choice of substances used for therapeutic formulations according to the present invention, it is preferred to use, if possible, natural products; anyway, in order to get more sophisticated characteristics to the finished product, synthetic substances were also taken into consideration.

So, a classic formulation is on castor oil basis, mainly constituted of triglyceride of castor fatty acid, that reacts through its hydroxylic groups with isocyanate groups of isocyanate polymer.

Also in this case, the formulation is constituted of two components to be mixed at the use moment, namely component A having hydroxilic aminic or amidic functions, and B having isocyanate functions.

Component A can be formulated with synthetic products, obtained through reaction of polyvalent alcohols with mono- or polycarboxylic acids, preferably of aliphatic or cycloaliphatic series. The product, synthesized through polycondensation, has a molecular weight preferably between 500 and 2000 and a medium-low viscosity, in order to allow a lowest use of organic solvents. This component A can also be obtained from natural oils, prereacted with polyvalent alcohols, with consequent formation of hydroxylated glycerides successively reacted with carboxylic acids. Natural oils, mostly taken into consideration for such purpose, are the following: linseed, castor, soya, grape-stone, maize, safflower, sunflower, groundnut, fish, oiticica, tung, perilla, cotton-seed, olive, almond, walnut, hazel, coco-nut, palm oils, and others of vegetable or animal derivation, all included into the chemical family of triglycerides of fatty saturated and unsaturated acids.

As polyvalent alcohols, there can be used glycols such as ethylenic, propylenic etc. and their polyoxyalkylenic derivatives, polyalcohols such as glycerin, trimethylolpropane, pentaerythritol, hexanetriol, sorbitol, hexoses and saccharose. Monovalent alcohols can also be used as modifying agents.

Monocarboxylic synthetic or natural aliphatic acids can be used as carboxylic acids, such as fatty, bi- and polycarboxylic acids, such as succinic, adipic, etc. phtalic, isophthalic, terephthalic, tetrahydrophthalic, hexahydrophthalic, and endomethylene-tetrahydrophthalic acids. Hydroxy acids, amino acids or lactones can also be included into the formulation of component A.

Component A can be also constituted of a hydroxylated urethane copolymer obtained through reaction of polyvalent alcohol, optimally modified by esterification or copolymerization with a polyisocyanate.

Component A can also be a copolymer obtained from unsaturated monomers having OH alcoholic or phenolic groups, amidic or aminic groups as residual functions.

Analogously, component A can also be a polyamide or polyesteramide, preferably having a low molecular weight, or a polyether obtained through condensation of a polyvalent alcohol with alkylene

oxides or chlorohydrin, or still further a polyamine with aminic secondary hydrogens, preferably sterically hindered.

Component A can also be constituted of lactone polymers.

As to the formulation of component B, it is principally characterized by the presence of isocyanate radicals (—NCO). Among substances that have these requirements, are included isocyanate oligomers, obtained by addition of a polyol with isocyanates such as isophorone diisocyanate, trimethylhexamethylene diisocyanate, hexamethylene diisocyanate, dicyclohexylmetane diisocyanate. Though less suitable, other substances of this kind are the addition products or oligomers based on aromatic isocyanates, such as toluene diisocyanate, diphenylmethane diisocyanate. As component B, there can be used allophanate trimers of the above-mentioned aliphatic or cycloaliphatic isocyanates, and also isocyanurate trimers thereof; and allophanate or isocyanurate copolymers of isocyanates, or even mixtures of pure trimers or copolymers should be included.

Component B can also be constituted of an addition product obtained by reaction of diisocyanates with a secondary diamine or polyamine or with a primary monoamine in suitable stoichiometric ratios.

Since the essential characteristic for the expression of the therapeutic properties of products according to the present invention is the presence of isocyanate groups, to which the capacity of chemically binding with skin proteins is attributed, component B can also be the sole component, provided that it has the chemico-physical-mechanical characteristics suitable to the task of skin protection.

Anyway, the formulation of the proudct with two components to be mixed at the application moment, offers more possibilities in modifying the characteristics, because it allows the addition of modifying additives such as catalysts, solvents, viscosity regulators, opacifying agents, dyes, permeability agents, auxiliary drugs with percutaneous action, to component A which is chemically more stable. Additives belonging to the formulation of component A, are important because they help to improve the product performances. So, in order to fit the film appearance to the natural one of skin, to satisfy aesthetic requirements, there can be used natural or synthetic waxes, microdispersed in suitable solvents in the state of micropowder; paraffins with selected melting point; powdery opacifying organic agents, such as polyethylene, polypropylene, polytetrafluoroethylene or similar copolymers, inorganic opacifying agents of silicic nature or on carbonate basis, preferably of calcium and magnesium, or on aluminium hydroxyde or oxide basis.

The solvents optionally used are chosen among those that have the best chemico-physical compatibility for skin, and should have a volatility as high as possible, so as to remain in contact with the treated part for the shortest possible time. Auxiliary drugs that can be used, must be selected among the inert ones toward the isocyanate groups, in order to avoid inactivation, or they must anyhow be used in an active form, free from functions that interfere in the reaction with the polymer. Particularly when the polymer is used for the transdermal absorption of drugs, there are employed as carriers substances having solvent function, such as triacetine (glycerine triacetate), dimethylsulfoxide, butyl stearate, ethyl or methyl linoleate, methylpyrrolidone, ethyl caprylate or other higher esters or solvents suitable for the treatment. As agents enhancing the permeability to the air of the applied films, there can be considered hydrophylic fibrous fillers or water-soluble polymers dissolved in an organic solvent not reacting with isocyanates.

Since the most easily commercially available isocyanates are characterized by some residual volatility making them irritating for the respiratory organs, as essential constituents of component B, there can be used addition compounds or polymeric derivatives of these isocyanates or alternatively pure di- or polyisocyanates with a relatively high molecular weight obtained in a synthetic way, in order to avoid the volatility problems. The present invention will now be particularly shown on the basis of some embodying examples, not given in a limitating sense.

*Component A*

Example 1

876 parts of adipic acid, 540 parts of 1,4-butanediol, 174 parts of 1,10-decanediol are reacted at 200°C in conditions of controlled reaction, so as to eliminate the condensation water (13.5% on the whole), by avoiding a loss of reagents, to obtain a polyester having an acidity index (mg of KOH/g of product) lower than 1, and a hydroxylic number = 89.

Example 2

523 parts of decanediol, 404 parts of sebacid acid, and 0.4 parts of tin dibutil dilaurate are reacted under the conditions of Example 1, until there is obtained a polyester having an acidity index = 0.5 and hydroxylic number = 135.

Example 3

536 parts of polyethoxylated glycerin (molecular weight = 268), 116 parts of fumaric acid, 138 parts of salicylic acid, and 1.5 parts of tin dibutyl dilaurate are reacted under conditions of Example 1, to obtain a polyester having a condensation degree = 99.9%.

Example 4

890 parts of soya-bean oil, 140 parts of pentaerythritol and 0.3 parts of stannous chloride are reacted at 235°C with the formation of a monoglyceride. The obtained monoglyceride is then reacted with 146 parts of adipic acid at 200°C until a polyester is obtained having an acidity index = 1 and hydroxylic number = 98.

Example 5

Example 4 is repeated with the difference that soya-bean oil is replaced with the same quantity of dehydrated castor oil. A polyester having analogous characteristics is obtained.

Example 6

900 parts of fish-oil (sardine oil), 184 parts of glycerin and 1 part of tin dibutyl dilaurate are reacted at 235°C in order to obtain the oily monoglyceride. This is cooled to 50°C and reacted with 420 parts of trimethyl hexamethylene diisocyanate to obtain a polymer having an isocyanic number = 0, and hydroxylic number = 75.

Example 7

174 parts of 1.10-decanediol, 938 parts of castor oil and 222 parts of isophorone diisocyanate are reacted as in Example 6 to obtain a polymer having isocyanic number = 0 and hydroxylic number = 114.

Example 8

158 parts of trimethylhexamethylendiamine, 280 parts of oleic acid and 300 parts of hydroxystearic acid are reacted at 200°C to obtain a polyamide having an acidity index = 1, hydroxylic number = 80 and hydrogen amide equivalent = 350.

Example 9

There are reacted 134 parts of trimethylol propane and 1068 parts of propylene oxide in the presence of 0.03 parts of metallic sodium at 5 Atm (490,000 Pa) pressure, and 140°C temperature to obtain a polyether having a hydroxyl index 140. At the end of reaction, the polyether is neutralized with 0.3 parts of magnesium hydrogen phosphate, $Mg(H_2PO_4)_2$ and is filtered off to obtain a limpid product.

Example 10

The following starting compounds are used to prepare a prepolymer A (the figures are parts by weight):

| | |
|---|---|
| Castor oil | 938 |
| Adipic acid | 438 |
| 1,10-decanediol | 174 |
| 1,6-hexanediol | 236 |
| | 1,786 |

In a reaction vessel suitable for effecting esterification the castor oil, adipic acid, 1,10-decanediol and 1,6-hexanediol are charged keeping the atmosphere of the reactor under nitrogen stream. The mixture is esterified by heating, mixing the ingredients to the temperature of 200°C under conditions of azeotropic reflux, using toluene as carrier solvent. In this way, the reaction-water is extracted and separated continuously from toluene in a suitable phase separator. In the rectification column, the temperature of the vapors at head of column is controlled so as not to exceed 100°C. The vapors of reactions are condensed in a water-cooled condenser. The reaction is continued until the complete extraction of the theoretical reaction water (108 g) and until the acidity value of the prepolymer is equal or less than 0.5 and the hydroxyl number equal to 92.

The toluene is distilled under vacuum of 10 torr (1,333 Pa) and the product is cooled to 50°C. The thus obtained product can be better transformed in component A by adding catalysts like tin dibutyl dilaurate (0.05—1%) alone or together with a tertiary aliphatic amine such as triethylamine (0.05—0.5%) and a solvent like acetone in order to give to the final product a reactivity and flow degree more suitable to the specific application.

*Component B*

Example 11

510 parts of 1,6-hexamethylenediisocyanate are reacted with 18 parts of water in conditions known per se, in order to obtain at the end of reaction a biuret structured polymer with a NCO content equal to 24% and hexamethylenediisocyanate monomeric content less than 0.2%.

Example 12

168 parts of 1,6-hexamethylenediisocyanate, 222 parts of isophorone diisocyanate, 210 parts of trimethylhexamethylenediisocyanate and 18 parts of water are reacted in conditions known per se, in order to obtain at the end of reaction a biuret structured polymer with a content of NCO functions equal to 20% and a content of monomeric diisocyanate less than 0.3%.

Example 13

440 parts of isophorone diisocyanate and 210 parts of trimethylhexamethylenediisocyanate are reacted in the presence of 0.2 parts of sodium methylate under conditions known per se, in order to obtain an isocyanurate polymer having a NCO content equal to 16.8% and a diisocyanate monomeric content less than 0.3%. The obtained polymer is diluted with anhydrous ethyl acetate and the sodium ion is eliminated with 1.2 parts of orthochlorobenzoyl chloride, and filtering on ultraanhydrous paper.

Example 14

Through addition reaction of 174 parts of 1,10-decanediol with 420 parts of trimethylhexamethylenediisocyanate in conditions known per se, there is obtained a product having a content of NCO functions equal to 13.8% and free isocyanate monomeric content less than 0.3%.

Example 15

212 parts of myristylamine are reacted using conditions known per se with 420 parts of trimethylhexamethylenediisocyanate and at the end of reaction there is obtained a product having a percentage of NCO function equal to 12.6% and a free monomeric isocyanate content less than 0.3%.

Example 16

By reacting 420 parts of trimethylhexamethylenediisocyanate and 228 parts of diisobutylhexamethylenediamine under conditions known per se, at the end of reaction there is obtained a product having a percentage of NCO functions equal to 12.3% and content of free monomeric isocyanate less than 0.2%.

The product thus obtained has been employed in the treatment of skin afflictions according to the present invention.

Example 17

The following starting substances are used to prepare a prepolymer B:

| | |
|---|---|
| Trimethylhexamethylenediisocyanate | 210 parts/wt |
| Hexamethylenediisocyanate | 336 |
| 1,10-decanediol | 130 |
| Distilled water | 9 |
| Ethyleneglycoldimethylether | 670 |
| Acetone | 75 |
| | 1,430 |

In the reaction vessel are charged trimethyl hexamethylenediisocyanate and hexamethylenediisocyanate. The reactor is purged with anhydrous nitrogen and heated to the temperature of 80°C.

There is prepared a solution mixing 1,10-decanediol water ethyleneglycol dimethylether and this solution is added gradually in 8 hours keeping the temperature in the reactor at 80°C.

Solvent vapours of ethyleneglycol dimethylether are condensed in a water cooled condenser and recycled in the reactor.

After completion of the addition of solution, the product is kept in the reactor always at 80°C. It is then distilled by heating gradually at 100°C recovering the solvent.

A vacuum of 10 Torr (1,333 Pa) is applied to the reactor while keeping the temperature at 100°C, inserting between the reactor and the vacuum pump an extra condenser and recovery tank overcooled at −35°C. These conditions of vacuum are kept for 30 minutes. The vacuum is stopped and the atmosphere in the reactor is saturated with nitrogen.

The prepolymer thus obtained is treated continuously in a thin-layer-evaporator under vacuum of 0.1 Torr (13.3 Pa) and with evaporator wall temperature of 125°C. This treatment is done to reduce to the minimum the content of free diisocyanate monomer. The prepolymer is cooled to 40°C and diluted with anhydrous acetone.

The final product contains reactive NCO groups in a percentage of 15.4% and a content of free diisocyanate monomers of 0.3%.

Combinations of Component A + B

Example 18

To 90 parts of the polyester obtained according to Example 1, are added 9.8 parts of ethyl acetate, 0.1 parts of paraffin having a melting point of 90°C and 0.1 parts of tin octate, in order to obtain a finished product corresponding to component A according to the present invention. This component A is mixed at the moment of use with the biuret polymer obtained in Example 11 in a weight ratio between 3:1 and 5:1 for the treatment of skin afflictions.

Example 19

99 parts of the polymer obtained according to Example 9 and 1 part of tin dibutyl dilaurate are combined at the application moment with the isocyanurate polymer obtained in Example 13 in a weight ratio varying between 1:1 and 3:1.

Formulations so obtained have been used in the treatment chronical eczema, lasting for 2—5 years and judged incurable because the cures, executed before on the basis of liniments and preparations according to the modern pharmacology, did not give any tangible result, while cortisone medicines desultorily used, sometimes provided improvements, but the pathology again occurred with recrudescence.

The application of the product according to the present example, caused soft films to be formed, that keep unimpaired for a variable period of time, ranging from about 7 to 20 days, according to cases, showing a remarkable resistance to water and to wear and tear, particularly when applied to the hands. With the film detachment because of the renovation of the skin horny layer, good results were mostly obtained. In the few cases in which the result was not resolutive, a second treatment or eventually a third one, in the most difficult cases, determined the complete healing.

Example 20

95.65 parts of anhydrous castor oil, 0.05 parts of lauryl amine, 0.2 parts of tin dibutyl dilaurate, 0.1 parts of paraffin with melting point 48°C, 2 parts of beta-carotene, and 2 parts of powdered polyethylene having a maximum particle size of 50 micron, are combined at the application moment with a product obtained through trimerization of the trimethylhexamethylenediisocyanate (trimer allophanate), according to respective weight ratios included between 1:1 and 2.5:1 for the treatment of skin afflictions.

Example 21

91 parts of the polymer obtained according to Example 7, 10 parts of N-vinyl pyrrolidone, 1 part of benzoinethylether and 1 part of tin dibutyl dilaurate are mixed at the application moment with the allophanate polymer according to Example 12 in respective weight ratios included between 2:1 and 4:1 for the treatment of skin afflictions.

Example 22

99 parts of the product obtained according to Example 6, 0.5 parts of cobalt octoate, and 0.5 parts of calcium octoate, are combined at the application moment with the isocyanurate polymer according to Example 13, preferably in a weight ratio 2:1 respectively for the treatment of skin afflictions.

Example 23

Combination of products according to Examples 1 to 10 with products according to Examples 11 to 17, are used to cure eczema, dermatitis, mycotic afflictions, and bedsores, with positive resolutive results after 20 days from the starting of the treatment.

Example 24

A combination of products according to Example 18, was used in order to treat a mycosis form that appeared as whitish plaques on the patient's legs.

The combination was applied immediately after the mixing of components A + B with a brush in order to cover completely the infested parts (about 150 $cm^2$ on the whole). After about 4—5 days the applied polymer film spontaneously peeled off, leaving a skin surface lightly reddened. The polymer application was successively repeated and the resulting film kept adhered to the skin for a much longer period of time and detached after about 15 days. At the end, the treated part was completely healed and there were no relapses.

Example 25

A combination of components according to Example 20, was applied to the left zygoma temporal part of a patient afflicted with a skin alteration of unsure diagnosis (psoriasis or lupus). 20 minutes after the application the patient already felt a beneficient sensation of moderate warmth at the site of application which replaced the burning sensation.

After 40 minutes, the cross-linked film was dry, with perfect elasticity which did not give any unpleasant sensation of superficial stretching. In the meantime, spasms due to superficial nervous contractions that, before the treatment, determined local microwounds, disappeared. The detachment of the polymer film happened after about 20 days. There resulted immediately the suspension of ache, and

the diminution of the red livid colouring. A second application was made and, after another 15 days, the polymer layer detached. The underlying skin surface was rose-coloured, typical of skin in a healing phase. At the end, there was executed a third application, to which the reconstitutive phase of skin followed. The doctor judged that the reconstituted skin had an appearance really better than the one regarding analogous cases of healings, obtained with radiotherapic treatments, combined with applications of specific medicines.

With reference to this, it is very important to note that the soothing action against the inflammatory symptoms, due to the reactive polymers according to the present invention, suggests anti-histaminic properties in the treatment of the infection. In fact the isocyanate radicals chemically block and neutralize the histamine molecules released by the tissues in concomitance with pathological phenomena, by binding the aminic groups belonging to them.

Example 26

A case of skin pathology, in which the formation of superficial chaps appeared, more properly of rhagades on the palmar surface and between the fingers and on the plantar feet part, was treated with a combination of products according to Example 20, and the condition was alleviated in short time. Immediately after the application of the preparation the classic rhagades burning disappeared; hands were kept with fingers wide apart and feet were kept free from the contact with foreign bodies for 30 minutes, until a fair degree of dessication was achieved. After 3 days, through the transparent polymer film, there could be already observed the complete cicatrization of the wounds.

Example 27

Some combinations of products according to Examples 18, 20, 21, 22 and 24, were used for the treatment of hands and feet chilblains (erythema pernio) with very good results, shown by the neutralization of skin irritation consequent to the particular pathology. After 15—20 days, the treatment was finished with perfect healing.

Example 28

A combination of products as reported in Example 22 was used for the cure of bedsores at the sacral bodily part of an old sick woman.

Previously, the woman had been treated with frequent applications of siliconic products without any positive result, but with progressive extension of the wounded part.

The application of the product according to the present invention was executed after having well dried the part; the patient immediately felt a great benefit, so that she was able to lean on the part with more facility without feeling great pain; since the previous siliconic applications partially compromised the film adhesion according to invention it was necessary to repeat the application 5 days later.

The bettering was considerable, but because of the compromising of the part due to the previous treatment, it was necessary to apply a third treatment with the products of the present invention, which was effected 15 days after the detachment of the second polymer film. At the end there was obtained the perfect healing of the wounded part.

Example 29

There is prepared a type of component A by mixing 60 parts of the product according to Example 7, 0.2 parts of paraffin (melting point 50°C), 20 parts of glycerine triacetate, 8 parts of glycerine trinitrate, 1 part of tin dibutyl dilaurate, 0.2 parts of triethylamine, 10 parts of ethyl caprylate and 2.6 parts of pyrogenic silica. The product, obtained in such a way, was thixotropic.

4 parts of this component A were combined, by mixing, with 1 part of component B, prepared as shown in Example 11. This combination was spread to a thickness of 1000 micron and a surface of 30 $cm^2$, on the back of a patient subjected to therapy for angina pectoris. This treatment showed an efficacy protracted for 4 days, that is for all the time the film remained attached to skin. Therefore, it was not necessary to repeat the application every day as is often required when a transdermal ointment is used. Besides, there was the advantage due to the possibility of doing the cleaning without removing the medicament supply in situ.

Example 30

There was prepared a type of component A with 80 parts of product according to Example 7, 0.5 parts of tin dibutyl dilaurate and 18.5 parts of allyl acetylsalicylate. This preparation A was combined with component B in the ratio 3:1 (as shown in Example 11). This product was spread to a thickness of 500 microns and a surface of 50 $cm^2$ on the internal part of the right thigh of a subject suffering from rheumatism. The film remained attached for 5 days. During this period of time, the application showed efficacy in considerably reducing the pain of articulations and eliminating the typical rheumatoid shivering sensations.

Example 31

There was prepared a type of component A with 98 parts of monodehydrated castor oil (that is with a ricinoleic radical transformed into a conjugated linoleic radical by eliminating one water molecule), 0.1 parts of tin dibutyl dilaurate and 1.9 parts of dimethylsulfoxide.

This component A was combined in the ratio 3:1 with component B (as shown in Example 11). The combination was applied on burnsores having a total surface of about 250 $cm^2$ on a patient's abdomen. Before the application, the necrotized tissues were removed and the part to be treated was hygienically prepared so that it showed the lowest exudation. The applied product kept oily consistency for about 1 hour. After about 2 hours, the part kept in contact with air under ventilation was almost dried. After 3 hours, a barrier film was formed protecting the treated part. About 15 days layer, the gradual detachment of the film together with the devitalized tissue surface occurred. The burnt surface was free from sores. A further application was made and, 10 days later, after complete polymer film detachment, the skin epitelial tissue of the treated part was perfectly reconstituted and healed.

Though the present invention has been explained on the basis of some embodying examples, it is obvious that variations and/or modifications can be introduced both in the description and examples.

**Claims**

1. Use of crosslinkable polyurethane resins with film-forming properties consisting of liquid isocyanate prepolymers alone or mixed with prepolymers having free hydroxy, amino or amido groups, possibly together with additives, adjuvants, solvents and transcutaneously acting drugs, to produce dressings against skin disorders and body diseases.

2. Use according to claim 1, characterized in that said dressings are formed at the moment of use by applying to the human or animal skin said liquid isocyanate prepolymer alone or instantly mixed with said prepolymers having free hydroxy, amino or amido groups.

3. Polyurethane resins for use according to claim 1, characterized in that said isocyanate prepolymers are oligomers having a content of free isocyanate (—NCO) groups ranging from about 12% to about 24%.

4. Polyurethane resins for use according to claim 1, characterized in that said prepolymers having free hydroxy, amino or amido groups are oligomers selected from the group consisting of polyesters, polyethers, polyamides, hydroxylated polyurethanes, and polyamines with low molecular weight.

5. Polyurethane resins for use according to claim 4, characterized in that said oligomers having free hydroxy, amino or amido groups have an acidity index from 0.5 to 1, hydroxy number between about 75 and 140 and a molecular weight between 500 and 2000.

6. Dressings for use according to claim 1, characterized in that the weight ratio between said prepolymers having free hydroxy, amino or amido groups and said isocyanate prepolymers is from 1:1 to 5:1.

**Patentansprüche**

1. Anwendung vernetzbarer Polyurethanharze mit Filmbildungseigenschaften bestehend aus flüssigen Isocyanatvorpolymeren allein oder vermischt mit freie Hydroxyl-, Amino-, oder Amidogruppen aufweisenden Vorpolymeren, gegebenenfalls zusammen mit Zusatzstoffen, Hilfs-, Lösungs- und transdermal wirkenden Arzneimitteln, zur Erzeugung von Verbandstoffe gegen Hautbeschwerden und Körperkrankheiten.

2. Anwendung gemäss Anspruch 1, dadurch gekennzeichnet, dass die genannten Verbandstoffe, indem man das genannte flüssige Isocyanatvorpolymer allein oder sofort mit dem genannten freie Hydroxyl-, Amino-, oder Amidogruppen aufweisenden Vorpolymer gemischt auf die menschliche oder tierische Haut auftragt, gebildet werden.

3. Polyurethanharze zur Anwendung gemäss Anspruch 1, dadurch gekennzeichnet, dass die genannten Isocyanatvorpolymeren Oligomeren mit einem Gehalt an freien Isocyanatogruppen (—NCO) zwischen ca. 12% und ca. 24% sind.

4. Polyurethanharze zur Anwendung gemäss Anspruch 1, dadurch gekennzeichnet, dass die genannten freie Hydroxyl-, Amino-, oder Amidogruppen aufweisenden Vorpolymeren Oligomeren aus dem Gruppe bestehend aus Polyestern, Polyethern, Polyamiden, hydroxylierten Polyurethanen und Polyaminen mit Niedermolekulargewicht sind.

5. Polyurethanharze zur Anwendung gemäss Anspruch 4, dadurch gekennzeichnet, dass die genannten freie Hydroxyl-, Amino-, oder Amidogruppen aufweisenden Oligomeren einen Säurewert von 0,5 zu 1, eine Hydroxylzahl zwischen 75 und 140 und ein Molekulargewicht zwischen 500 und 2000 haben.

6. Verbandstoffe zur Anwendung gemäss Anspruch 1, dadurch gekennzeichnet, dass das Gewichtsverhältnis zwischen den genannten freie Hydroxyl-, Amino-, oder Amidogruppen aufweisenden Vorpolymeren und den genannten Isocyanatvorpolymeren zwischen 1:1 und 5:1 liegt.

**Revendications**

1. Emploi de résines polyuréthane réticulables avec des propriétés de formation de film constituées par des prépolymères isocyanate liquides seuls on en mélange avec des prépolymères ayant des groupes hydroxyle, amino ou amido libres, le cas échéant avec des additives, adjuvants, solvants et médicaments à action transcutanée, pour la production de pansements contre les troubles de la peau et les maladies du corps.

2. Emploi selon la revendication 1, caractérisé en ce que lesdites pansements sont préparés au moment de l'emploi en appliquant à la peau de l'homme ou de l'animal ledit prépolymère isocyanate liquide seul ou mélangé à l'instant avec ledit prépolymère ayant des groupes hydroxyle, amino ou amido libres.

3. Résines polyuréthane pour l'emploi selon la revendication 1, caractérisées en ce que lesdits prépolymères isocyanate sont des oligomères ayant un contenu de groupes isocyanate (—NCO) libres compris entre 12% environ et 24% environ.

4. Résines polyuréthane pour l'emploi selon la revendication 1, caractérisées en ce que lesdits prépolymères ayant des groupes hydroxyle, amino ou amido libres sont des oligomères choisis dans le groupe constitué par polyesters, polyethers, polyamides, poliurethanes hydroxylés et polyamines à poids moleculaire bas.

5. Résines polyuréthane pour l'emploi selon la revendication 4, caractérisées en ce que lesdits oligomères ayant des groupes hydroxyle, amino ou amido libres ont un index d'acidité de 0,5 à 1, un nombre d'hydroxyle entre 75 et 140 et un poids moléculaire entre 500 et 2000.

6. Pansements pour l'emploi selon la revendication 1, caractérisés en ce que le rapport en poids entre lesdits prépolymères ayant des groups hydroxyle, amino ou amido libres et lesdits prépolymères isocyanate est compris entre 1:1 et 5:1.